Europäisches Patentamt

European Patent Office

Office européen des brevets

(11) Publication number: **0 652 289 A1**

# EUROPEAN PATENT APPLICATION

(21) Application number: **93308864.3**

(22) Date of filing: **05.11.93**

(51) Int. Cl.6: **C12P 7/64**, C11C 3/10, A23D 9/00

(43) Date of publication of application:
**10.05.95 Bulletin 95/19**

(84) Designated Contracting States:
**AT BE CH DE DK ES FR GB GR IE IT LI LU MC NL PT SE**

(71) Applicant: **UNILEVER PLC**
**Unilever House**
**Blackfriars**
**London EC4P 4BO (GB)**

(72) Inventor: **Moore, Stephen Raymond c/o**
**Unilever Research**
**Colworth Laboratory**
**Colworth House**
**Sharnbrook**
**Bedford**
**MK44 1LO (GB)**
Inventor: **Ouinlan, Paul Thomas c/o Unilever**
**Research**
**Colworth Laboratory**
**Colworth House**
**Sharnbrook**
**Bedford**
**MK44 1LO (GB)**
Inventor: **Peilow, Anne Cynthia c/o Unilever**
**Research**
**Colworth Laboratory**
**Colworth House**
**Sharnbrook**
**Bedford**
**MK44 1LO (GB)**

(74) Representative: **Sikken, Antonius H. J. M. et al**
**UNILEVER N.V.,**
**Patent Division,**
**P.O. Box 137**
**NL-3130 AC Vlaardingen (NL)**

(54) **Random interesterification of triglyceride fats.**

(57) Process for the preparation of a triglyceride mixture with an approximately random distribution of the fatty acid residues over the glyceride positions Sn1, Sn2 and Sn3, by subjecting in the presence of water a triglyceride fat to the activity of a 1,3-specific lipase.
The process is a multi-step process which includes a first step wherein the triglycerides are subjected to enzymatic action in the presence of diglycerides amounting 4-30 wt.% on total fat content and a second step wherein the diglyceride concentration in the reaction mixture is reduced, e.g. by removal of water, optionally after addition of fatty acids.

The present invention deals with an interesterification process of triglyceride fats. More particularly the process concerns an enzymatic interesterification process.

Interesterification processes of triglycerides are directed to an exchange of the fatty acid residues of the triglyceride molecules. In the resulting triglycerides the fatty acid residues have been substituted by different residues, originating from the same or different glyceride molecules or from free fatty acids that may be present in the reaction mixture.

The interesterification process needs a catalyst, which often is an alkali metal hydroxide or an alkali metal alkanolate, such as sodium methanolate. At equilibrium the exchange of fatty acid residues results in a statistically even (random) distribution of the fatty acid residues over the three carbon positions of the glyceride molecule.

Alternatively, a lipase enzyme may be used as a non-chemical catalyst. Some interesterification lipases, the 1,3-specific lipases to which many *Rhizopus* lipases belong, only catalyse reactions on the positions Sn1 and Sn3 of the glyceride molecule. The ester bond on the Sn2 position remains unaffected, with the result that the randomisation process is limited to the terminal positions of the glyceride molecule. It goes without saying that after a interesterification treatment which affects only the terminal fatty acid residues, triglyceride mixtures are obtained of a nature which is quite different from the fully randomised triglycerides known from chemical interesterification. A consequence is that the extensive knowledge on and experience with fully randomised interesterified fats can no longer be used.

Other enzymes, comprising only a few non-specific lipases including *Candida cylindracae* and *Arthrobacter* lipases, are able to catalyse the hydrolysis and subsequent reformation of all three ester bonds with the effect that the randomisation extends also to the Sn2 position (full randomisation). Processes for enzymatic interesterification processes are described in e.g. WO 91/08676 (KRAFT).

Present consumer preference shifts to food and food ingredients which have not been exposed to chemical treatments. Therefore a general need exists for non-chemical modification processes of triglyceride fats. For interesterification an enzymatic process is preferred, because it does not change the naturalness of the fat product. However, fully randomised fats can only be obtained with non-specific lipases, which generally are either not suited for large scale modification process and/or have not been approved for food manufacture. On the other hand suitable interesterification enzymes are 1,3-specific lipases but these give no access to fully randomised triglyceride fats.

## STATEMENT OF INVENTION

A process has been found for the preparation of a triglyceride mixture with a random distribution of the fatty acid residues over the glyceride positions Sn1, Sn2 and Sn3, by subjecting in the presence of water a triglyceride fat to the activity of a lipase to a randomisation level of at least 50%, characterised in that a 1,3-specific lipase is used.

Preferably, the process is a multi-step process which includes a first step wherein the triglycerides are subjected to enzymatic action in the presence of diglycerides amounting 4-30 wt.% on total fat content and a second step wherein the diglycerides concentration in the reaction mixture is reduced.

The process results in a randomisation at a level which is at least 50%, preferably at least 80% of the theoretically attainable randomisation level.

## DETAILS OF THE INVENTION

The randomisation treatment should be carried out with a glyceride mixture which contains elevated levels of diglycerides. Elevated is understood to be in relation to the level normally present in triglyceride fats. The amount should be 4-30 wt.% diglycerides on total fat content.

Such levels of diglycerides are preferably obtained by adjusting the water content to 0.1-1 wt.%, preferably 0.2-0.6 wt.% on total fat content. Water generates the diglyceride *in situ* by fat hydrolysis. However, increased diglyceride levels can also be obtained by direct addition or by enzymatic synthesis *in situ* from glycerol and fatty acids.

The first process step is discontinued when the desired level of randomisation is obtained. The second process step is completed, when the amount of diglycerides is reduced to the desired level, usually <4 wt.%.

Suitable reaction times for the first process step are 10-120 hours, preferably 10-40 hours.

The extent of randomisation resulting from the process is expressed as randomisation level. For defining randomisation level use is made of the occurrence of specific triglycerides in the treated fat, e.g. SSS (S is a saturated long chain fatty acid such as palmitic acid and stearic acid) or the occurrence of a specific fatty acid on Sn2 which occurrences can be easily determined using standard methods. The chosen occurrence is arbitrary with the proviso that it has a value (wt.%) at the start of the process (time = $t_0$) which is not identical with the random value. The random value can be derived from the fatty acids distribution using standard statistical calculations.

The level (%) of randomisation at time t can be calculated using the formula:

$$Randomisation\ (\%) = ((AAA_t - AAA_0) / (AAA_r - AAA_0)) * 100\%.$$

where

$AAA_t$ is actual AAA weight at time t

$AAA_0$ is actual AAA weight at time 0

$AAA_r$ is calculated AAA weight in a fully randomised triglyceride mixture.

Where AAA is a specific triglyceride class which can be determined by the HPLC/silver phase method.

For establishing the extent of randomisation generally GLC-FAME and 2-position analysis of the initial feed stock and of the reaction mixture is used according to methods to be described later in the examples section. For determining specific triglycerides other methods can be applied e.g. the GLC/carbon number method or the HPLC/silver phase method.

The esterification of glycerides is a process in equilibrium with their hydrolysis, so that removal of one of the reaction products or an increase of the concentration of a reactant shifts the equilibrium to the synthesis of triglycerides under consumption of diglycerides and fatty acids. Therefore the amount of diglycerides may be effectively reduced by removing of the water from the reaction mixture, preferably by evaporating off the water under reduced pressure. The temperature preferably is 60°-80°C. The water evaporation results in low concentrations of diglycerides and fatty acids. The second process step usually takes 5 - 60 hours.

By having increased the concentration of fatty acids when the water is removed the amount of diglycerides can be further reduced.

The enzyme used for carrying out the invention is a 1,3-specific lipase. Suitable lipases may be derived from a micro-organism chosen from the group comprising *Rhizopus*, *Rhizomucor*, *Humicola* or *Pseudomonas*. Although these enzymes affect predominantly the Sn1 and Sn3 positions, some hydrolysis activity on the 2-position may be noticed.

Either the enzyme or an isolate may be used, or a micro-organism containing the enzyme. The enzyme preferably is attached to a carrier such as Duolite™ or Accurel™.

*Rhizomucor mienei* lipase attached on Duolite™, denoted as SP392, is a suitable catalyst.

The amount of catalyst generally is 1-10 wt.% of the total fat content. The appropriate amount for each system can be easily determined. For SP392 generally 1-5 wt.% is used.

The first process step preferably is carried out at 50-80°C.

The process of the invention can be applied on all kinds of triglycerides, but is most appropriate for triglyceride fats in which there is an imbalance in the distribution of fatty acids over the Sn1,3 and the Sn2 positions by at least one of the oils involved in the interesterification.

Therefore the process is very suitable for palm oil and palm oil fractions which show such imbalance.

The most surprising feature of the invention is that 1,3-specific lipases can be used as dual purpose enzymes.

Depending on reaction parameters, namely the employed level of diglycerides and the reaction time, an interesterified triglyceride results which either is 1,3-randomised or 1,2,3-randomised. The invention provides a non-chemical, technical scale process for the preparation of fully randomised "natural" interesterified triglycerides. These enzymatically prepared fats were earlier not accessible in large amounts. Because the triglyceride composition of such fats are identical with known chemically interesterified fats, protocols for further processing are widely available.

The invention is also embodied in food products in which a fat is incorporated which is obtained by the process of the invention.

DESCRIPTION OF THE FIGURES

**Fig. 1** Shows the level (%) of proceeding randomisation of a blend of palm mid fraction and a StOSt rich fat at different times (hours) for a water content of 0.5 wt.% and and an oil/catalyst ratio of 20:1.

**Fig. 2** Shows the diglyceride concentration (%) as a function of interesterification time (hours) for a blend of palm mid fraction and a StOSt rich fat with a water content of 0.5 wt.% and an oil/catalyst ratio of 20:1.

**Fig. 3** Shows the level (%) of proceeding randomisation of a blend of a PPLa rich fat and a StOSt rich fat at different times (hours) for a water content of 0.5 wt.% and and an oil/catalyst ratio of 20:1.

**Fig. 4** Shows the diglyceride concentration (%) as a function of interesterification time (hours) for a blend of a PPLa rich fat and a StOSt rich fat with a water content of 0.5 wt.% and an oil/catalyst ratio of 20:1.

## EXAMPLES

General methods

Fame preparation for neutral triglyceride oils

(This procedure is suitable for fats with free fatty acid levels not exceeding 2% w/w).

Weigh 10mg of fat into a 7ml screw-top sample vial.

Add 1ml of Micro Methyl Ester Reagent*, cap and react on a hot block at 60°C for 5 minutes, with occasional shaking.

Allow to cool and add 2ml of distilled water and then 2ml of Iso-octane with careful agitation. Pipette off top layer into a small auto vial, cap.

This solution is now suitable for injection.

Fame preparation for triglyceride oils containing free fatty acids

Intended for preparation of total FAMEof free fatty acids/combined fatty acids in one.

These samples require a reagent that will methylate FFA and for most cases Saponification followed by reaction with Boron Trifluoride - Methanol reagent is the best choice.

Take 10mg of fat in a 7ml vial suitable for the hot block.

Add 1ml of 2% KOH in methanol solution (0.5M), cap and react on the hot block for 5 minutes at 60°C, making sure all the fat has dissolved (ie. saponified).

Allow to cool (1 minute) and add 1ml of $BF_3$/Methanol reagent (fume hood). Replace on the hotblock for 5 minutes.

Allow to cool. Add 2 ml of distilled water and then 2ml Iso-octane, gently invert to extract the FAME into the iso-octane layer. Pipette off upper iso-octane layer into a small auto vial. (If the iso-octane layer appears cloudy, it can be dried over anhydrous sodium sulphate.)

This solution is now suitable for injection.

Lipolysis

About 0.5g of the fat or oil to be analysed is dissolved in dichloromethane (1ml) and is passed through an alumina column (Brockman I alkaline, 2g), with additional solvent washings to remove free fatty acids and partial glycerides. The solvent is removed by evaporation (nitrogen, 50°C) to recover the triglyceride fraction for further analysis.

Ifthe sample is a liquid or a soft fat, 0.1g of the cleaned up sample is weighed into a vial (25ml). Ifthe sample is a hard fat then 0.05g of a liquid monoacid triglyceride (tri-C9, > 99%purity), is added to 0.05g of the sample in order to aid dissolution. Buffer solution (2ml), and bile salt solution (0.5ml) are added.

This mixture is then placed into a sonic bath set to 40°C for 5 minutes to ensure complete emulsification. In the case of a particularly hard fat, it may be necessary to carry out the emulsification step at a higher temperature (60°C). Ifthis is done, then be careful to cool the vial to 40°C before proceeding to the next stage.

Calcium chloride (0.005g), and lipase (0.02g) is then added and the mixture put back into the 40°C water bath and shaken for a further 15 minutes.

The length of time required for this stage will depend upon the fat being analysed, and may take some experimentation to determine. As a general rule, the monoglyceride recovery should be between 15 and 25% based on the triglyceride taken.

Hydrochloric acid (1ml of 1M), is then added to stop the action of the enzyme. An internal standard (0.01g of C17 monoglyceride > 99%,accurately known) is then added and mixed in.

This standard will be used to determine monoglyceride recovery following gas chromatography of fatty acid methyl esters from the 2-monoglyceride (C17 peak). The C17 monoglyceride is assumed to have similar extraction behaviour to the 2 - monoglycerides at this stage.

The lipid components are extracted from the mixture and dried by adding diethyl ether (25ml) to the vial, shaking well and transferring the contents to a conical flask, to which anhydrous sodium sulphate (25g) is added. Wash the vial with ether twice more and add to the flask.

After mixing and settling, the ether layer is decanted into a clean vial, and the solvent is blown down (nitrogen, 40°C).

Dichloromethane (up to 0.5ml) is added to the recovered fat, and this solution is transferred to the preadsorbent strip of a silica TLC plate (0.5mm thickness 20x20cm, Merck S60). A monoglyceride marker is spotted either side of the sample.

The plate is then transferred to a TLC tank. The solvent system used consists of hexane (40ml), diethyl ether (60ml), and formic acid (1ml). The plate is removed when the solvent front is about 1cm from the top of the plate, and is allowed to dry. It is then sprayed with dichlorofluorescein to reveal the monoglyceride band, which is carefully marked.

The monoglyceride band is scraped off of the plate, and the monoglyceride is extracted from the silica, using diethyl ether (5ml, shaking). The silica is removed by filtration, and the solvent blown down as before. The recovered monoglyceride is

* Micro Methyl Ester Reagent: 2 (0.3% KOH in MeOH) : 1 (60/80 Petroleum Spirit) by volume.

then methylated, and analysed (GCFAME/893).

The monoglyceride recovery, and FAME profile at the 2-position can now be calculated from the FAME data.

### GC Methods

**Column:**
30m x 0.53mm DB225 capillary ex J&W Scientific
**Injection:**
Perkin Elmer PTV on-column injection
**Detector:**
FID set at 260°C
**Carrier gas:**
Helium, typically 50KPa
Solvent retention adjusted to 0.5min
**Injector PTV programmed:**
0.0min 50°C
0.5min 250°C
3.5min 50°C
Auto injection, typically 0.2$\mu$l injection volume of 2mg/ml solutions.

### 1. Standard Program for Oils and Fats with Fatty Acids Ranging C8:0 to C24:0

Temperature program:
120°C - 200°C @ 8°C/min      [WB26]
200°C - 230°C @ 2°C/min

### 2. Milk Fats and Butyric Acid (C4:0) Content Fatty Acids C4:0 - C24:0

Temperature program:
50°C - 200°C @ 10°C/min      [WB22]
200°C - 230°C @ 2°C/min

### 3. Fast Isothermal Analysis of C14:0 - C24:0

Isothermal oven temperature:
220°C      [WB30]

### Example 1

Randomisation of a blend of palm mid fraction and a StOSt rich fat

25 g of a fractionated palm mid fraction were mixed with 25 g of a StOSt-rich fat and 2.5 g of *Mucor miehei* lipase supported on Duolite™. Water was added to the reaction mixture and adjusted to 0.5% (determined by Karl Fischer titration).

In step 1 of the process scheme the reactants were stirred at 70°C for 24 hours, in a jacketed vessel fitted with a paddle stirrer. Samples were taken at intervals and the SSS-content (weight) determined by silver phase HPLC (Jeffrey B.S.J.

JAOCS, Vol 68, No. 5, 1991) and the partial glyceride content determined by straight phase HPLC (Hammond E.W.J., Chromatography, 203, 397, 1981). Step 1 was continued until the formation of a randomised triglyceride mixture has proceeded to 70%, at which stage there were 11% diglycerides in the reaction mixture.

The diglyceride content was reduced in step 2 of the process scheme by keeping the mixture under reduced pressure at 70°C for 15 hours, resulting in removal of free water. At the end of step 2 the randomisation had proceeded to 82%, whilst the diglyceride content had been reduced to 3%.

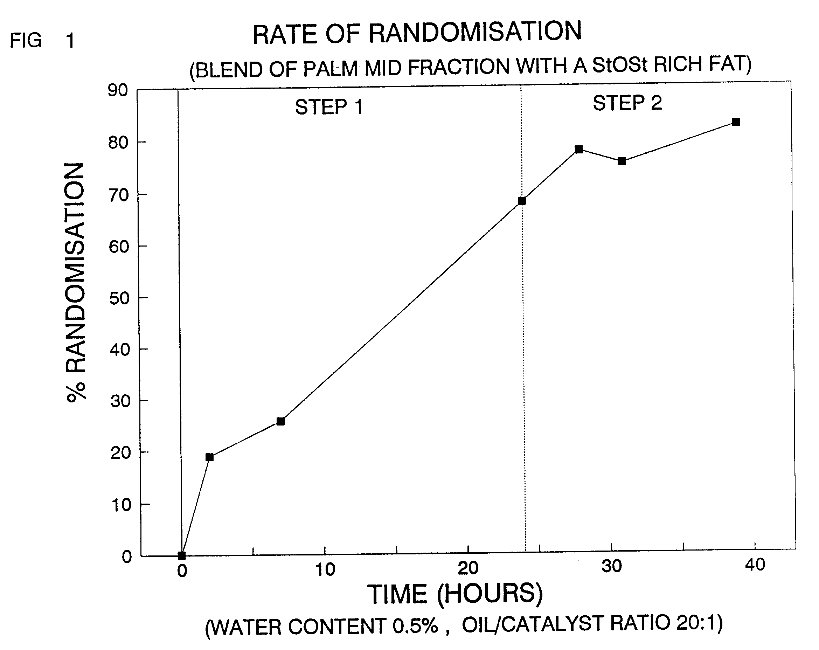

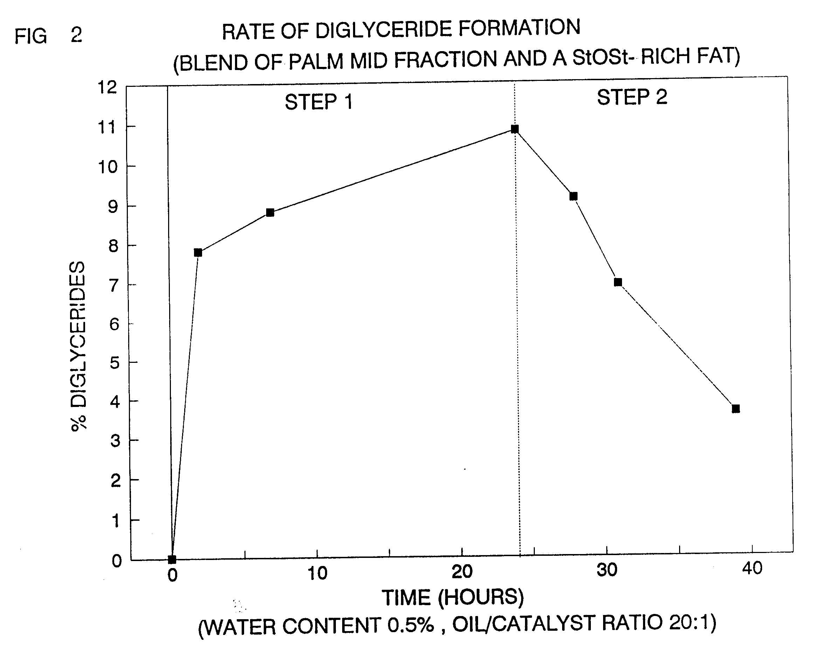

The degree of formation of randomised triglyceride as a function of time is illustrated in Fig 1 and the diglyceride content as a function of time in Fig 2.

The level (%) of randomisation at time t was calculated using the formula:

$$\text{Randomisation (\%)} = ((S3_t - S3_0) / (S3_r - S3_0)) * 100\%.$$

where
$S3_t$ is SSS weight at time t
$S3_0$ is SSS weight at time 0
$S3_r$ is calculated SSS weight in a fully randomised triglyceride mixture.

Where SSS is a triglyceride class which can be determined by the HPLC/silver phase method (S representing any long chain saturated fatty acid, in particular palmitic and stearic acids).

The actual SSS content in a fully randomised triglyceride mixture was calculated statistically using the formula:-

$$\text{\% SSS at completion of randomisation} = (\text{wt.\% long chain saturated acids})^3$$

The wt% of saturated acids was determined by FAME GLC by the method given above.

### Example 2

Randomisation of a blend of a PPLa rich fat and a StOSt rich fat

7.5 g of a fat rich in the triglyceride PPLa were mixed with 7.5 g of a StOSt-rich fat and 0.75 g of *Mucor miehei* lipase supported on Duolite™. Water was added to the reaction mixture and adjusted to 0.5% (determined by Karl Fischer titration).

The reactants were stirred at 70°C for 72 hours, in a magnetic stirrer/heater block. Samples taken at intervals and the 2-position of the triglycerides analysed by lipolysis/FAME GLC. The analytical methods used are described under the heading General methods. The level (%) of randomisation at time t was calculated using the formula:

$$\% \text{ Randomisation} = ((La^2_t - La^2_0) / (La^2_r - La^2_0)) * 100\%$$

where

$La^2_t$ is 2-position lauric acid content at time t

$La^2_0$ is 2-position lauric acid content at time 0

$La^2_r$ is calculated 2-position lauric acid content in a fully randomised triglyceride mixture.

In a fully randomised product the distribution of lauric acid at the 2 position should be the same as the overall lauric acid distribution.

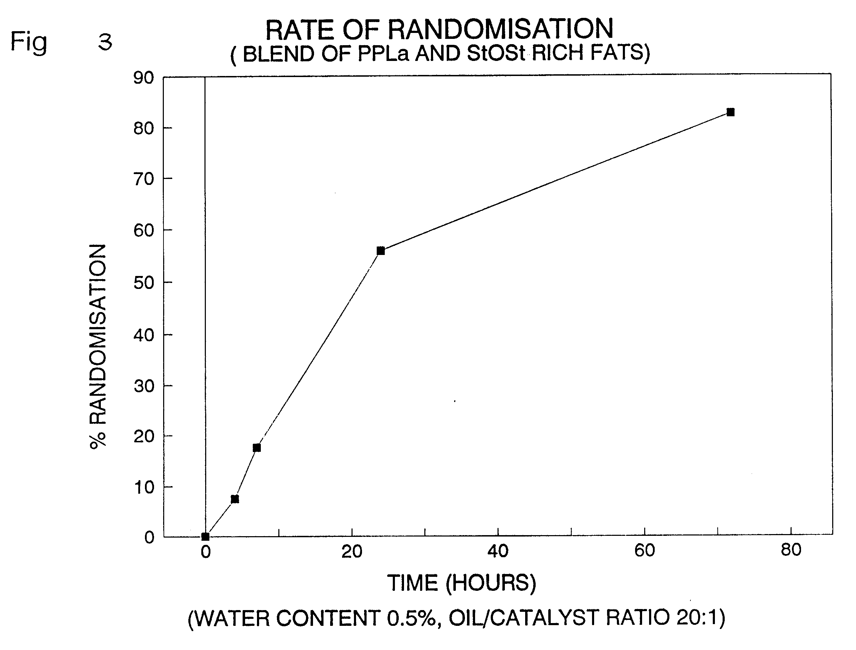

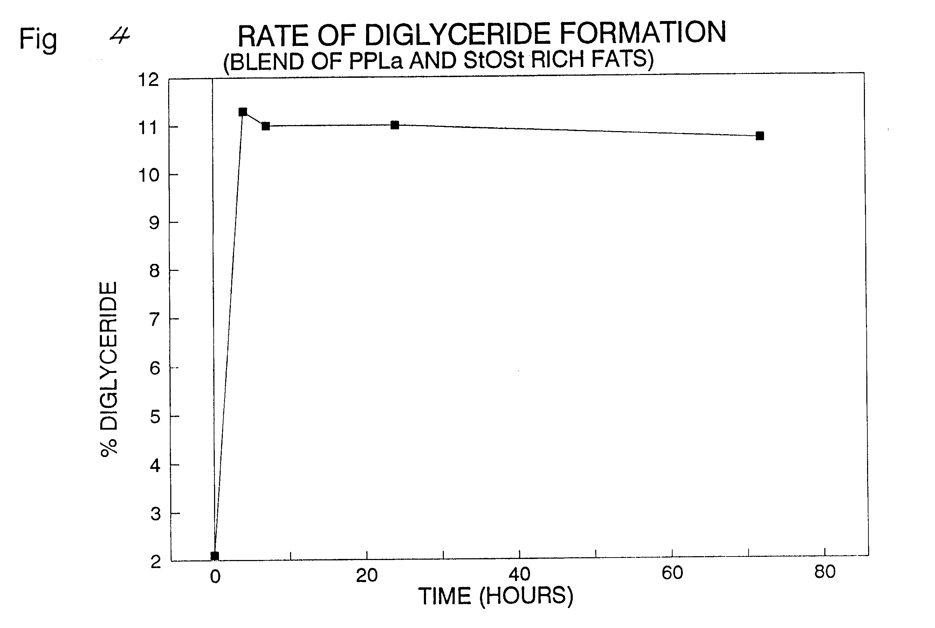

The degree of formation of randomised triglyceride as a function of time is illustrated in Fig 3 and the diglyceride content as a function of time in Fig. 4.

## Claims

1. Process for the preparation of a triglyceride mixture with a random distribution of the fatty acid residues over the glyceride positions Sn1, Sn2 and Sn3, by subjecting in the presence of water a triglyceride fat to the activity of a lipase to a randomisation level of at least 50%, characterised in that a 1,3-specific lipase is used.

2. Process according to claim 1, characterised in that the process is a multi-step process which includes a first step wherein the triglycerides are subjected to enzymatic action in the presence of diglycerides amounting 4-30 wt.% on total fat content and a second step wherein the diglyceride concentration in the reaction mixture is reduced.

3. Process according to claims 1 or 2, characterised in that the triglyceride mixture has a randomisation level of at least 80%.

4. Process according to any one of claims 1-3, characterised in that the triglyceride mixture contains water amounting to 0.1-1 wt.%, preferably 0.2-0.6 wt.% on total fat content.

5. Process according to any one of claims 2-4, characterised in that the second step comprises removal of the water in the reaction mixture resulting in a reduction of the amount of diglycerides.

6. Process according to claim 5, characterised in that the water is removed by evaporation under reduced pressure.

7. Process according to claims 5 or 6, characterised in that the water is removed after a preceding increase of the concentration of fatty acids.

8. Process according to any one of claims 1-7, characterised in that the enzyme is a 1,3-specific lipase derived from *Rhizopus*, *Rhizomucor*, *Humicola* or *Pseudomonas*.

9. Process according to any one of claims 2-8, characterised in that the first process step is carried out at a temperature of 50-80°C.

10. Process according to any one of claims 1-9, characterised in that the starting triglyceride mixture contains palm oil or a palm oil fraction.

11. Food products in which a fat is incorporated which is obtained by a process according to any one of the preceding claims.

FIG 1

## RATE OF RANDOMISATION
### (BLEND OF PALM MID FRACTION WITH A StOSt RICH FAT)

STEP 1    STEP 2

% RANDOMISATION

TIME (HOURS)

(WATER CONTENT 0.5% , OIL/CATALYST RATIO 20:1)

EP 0 652 289 A1

FIG 2    RATE OF DIGLYCERIDE FORMATION
(BLEND OF PALM MID FRACTION AND A StOSt- RICH FAT)

(WATER CONTENT 0.5% , OIL/CATALYST RATIO 20:1)

EP 0 652 289 A1

Fig 3

RATE OF RANDOMISATION
( BLEND OF PPLa AND StOSt RICH FATS)

% RANDOMISATION

TIME (HOURS)

(WATER CONTENT 0.5%, OIL/CATALYST RATIO 20:1)

EP 0 652 289 A1

Fig 4

## RATE OF DIGLYCERIDE FORMATION
### (BLEND OF PPLa AND StOSt RICH FATS)

EP 0 652 289 A1

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | ACTA CHEMICA SCANDINAVICA. SERIES B - ORGANIC CHEMISTRY AND BIOCHEMISTRY vol. 45, no. 7 , 1991 , COPENHAGEN DK pages 723 - 730 GUDMUNDUR G. HARALDSSON ET AL. 'Studies on the positional specificity of lipase from Mucor miehei during interesterification reactions of cold liver oil with n-3 polyunsaturated fatty acid and ethyl ester concentrates.' | 1 | C12P7/64 C11C3/10 A23D9/00 |
| Y | * the whole document * | 6-8,10 | |
| X | EP-A-0 245 076 (UNILEVER) 11 November 1987 * page 2, column 55 - page 3, column 5; claims * | 1 | |
| Y | FR-A-2 340 979 (UNILEVER) 9 September 1977 * claims * | 8,10 | |
| Y | WO-A-83 03844 (UNILEVER) 10 November 1983 * claims * | 8,10 | TECHNICAL FIELDS SEARCHED (Int.Cl.6) |
| Y | EP-A-0 170 431 (UNILEVER) 5 February 1986 * claims * | 8,10 | C12P C11C A23D |
| Y | EP-A-0 257 388 (KAO CORPORATION) 2 March 1988 * claims * | 6,7 | |

-/--

The present search report has been drawn up for all claims

| Place of search | Date of completion of the search | Examiner |
|---|---|---|
| THE HAGUE | 20 April 1994 | Delanghe, L |

EPO FORM 1503 03.82 (P04C01)

European Patent
Office

**EUROPEAN SEARCH REPORT**

Application Number

EP 93 30 8864

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | FETT WISSENSCHAFT TECHNOLOGIE- FAT SCIENCE TECHNOLOGY vol. 93, no. 2 , February 1991 , LEINFELDEN ECHTERDINGEN DE pages 41 - 49 XP176891 VON T.LUCK ET AL. 'Lipasekatalysierte triglyceridumesterung in einem lösungsmittelfreien prozess .I:Analytik und umesterungskinetik.' * the whole document * | 1 | |

TECHNICAL FIELDS
SEARCHED      (Int.Cl.6)

The present search report has been drawn up for all claims

| Place of search | Date of completion of the search | Examiner |
|---|---|---|
| THE HAGUE | 20 April 1994 | Delanghe, L |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)